# EUROPEAN PATENT APPLICATION

(11) **EP 4 596 673 A1**
(43) Date of publication of application: **06.08.2025**
(21) Application number: 23870586.7
(22) Date of filing: 21.09.2023
(51) Int. Cl.: C12N 5/00, C12N 1/02, C12M 1/00

(54) **MAGNETIC BEAD REMOVING METHOD AND DEVICE AND STORAGE MEDIUM**

(30) Priority: 27.09.2022 CN 202211181849
(71) Applicant: Shenzhen Cellbri Bio-Innovation Technology Co., Ltd., Shenzhen, Guangdong 518107 (CN)
(72) Inventor: GUO, Xiaoliang, Shenzhen, Guangdong 518107 (CN); SHANG, Yuanfang, Shenzhen, Guangdong 518107 (CN); ZHANG, Jinxin, Shenzhen, Guangdong 518107 (CN); LIU, Fujing, Shenzhen, Guangdong 518107 (CN); LI, Xiaosong, Shenzhen, Guangdong 518107 (CN)
(74) Representative: Herrero & Asociados, S.L.
(86) International application number: PCT/CN2023/120445
(87) International publication number: WO 2024/067354

(57) **Abstract**

The present disclosure discloses a magnetic bead removal method, device, and storage medium, including: placing a liquid bag with a curved flow channel on a magnetic platform, wherein a plane where the curved flow channel is located is above the magnetic platform and parallel to the magnetic platform; controlling a first flow control assembly to communicate the liquid bag with a sample container, thereby inputting a liquid containing immunomagnetic cells and non-magnetic immune cells into the curved flow channel from the sample container; performing a flattening operation on the liquid bag to increase a contact surface between a bottom surface of the curved flow channel and the magnetic platform, thereby adsorbing the immunomagnetic cells in the curved flow channel onto the contact surface through the magnetic platform; and performing a collection operation to collect the non-magnetic immune cells in the curved flow channel into a collection container. The present disclosure improves the uniformity of the flow velocity of the liquid in the liquid bag, enhances the utilization efficiency of the magnetic field, and achieves efficient and stable capture of the immunomagnetic cells.

## Description

The present disclosure claims priority to a Chinese patent application with application number 202211181849.4, titled "Magnetic Bead Removal Method, Device, and Storage Medium," filed on September 27, 2022, the entire contents of which are incorporated herein by reference.

### TECHNICAL FIELD

The present disclosure relates to the field of cell separation technology, and more particularly, to a magnetic bead removal method, device, and storage medium.

### BACKGROUND

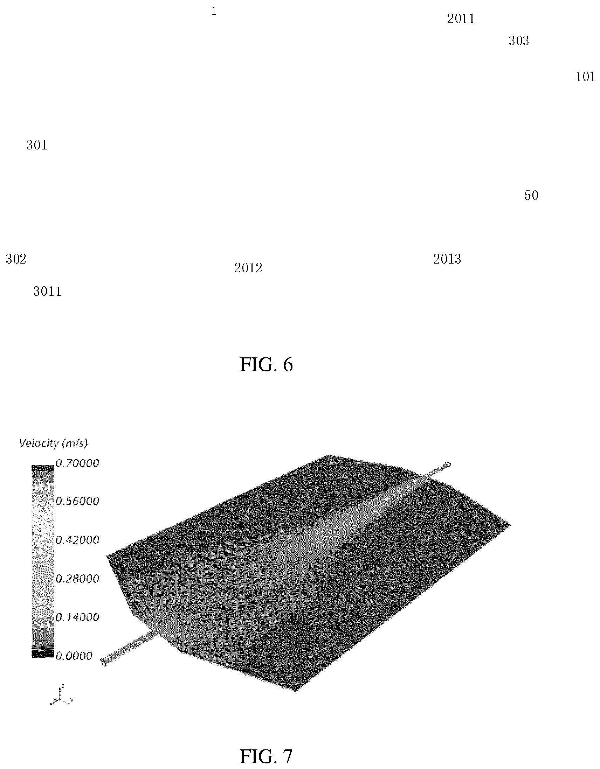

In the field of biotechnology, magnetic beads are often used to sort specific cells and bacteria, and then sorted cells or bacteria are cultured for experimental or medical purposes. At present, after micrometer sized magnetic beads are used to sort immunomagnetic cells and non-magnetic immune cells are cultured through the immunomagnetic cells, the immunomagnetic cells may still remain in the culture medium. Sometimes, the magnetic beads need to be removed by magnetic field adsorption to avoid adverse effects on experiments or medical treatment in the next step. The inventor realized that in the current technology, during the removal process of the immunomagnetic cells, due to the shear resistance of the fluid, when the area of the liquid bag containing the culture medium is too large (with the development of the cell processing industry, the volume of the sample in the liquid bag containing the culture medium is increasing, such as 5 L and 10 L), and the flow rate of the liquid bag containing the culture medium passing through the magnetic field is extremely uneven (as shown in FIG. 7), thus, the immunomagnetic cells in the area with excessive flow rate may escape, preventing the immunomagnetic cells from being fully adsorbed by the magnetic field and reducing the magnetic field utilization efficiency. To address the above issues, the flow rate of the cell fluid can be slowed down to reduce the escape of the immunomagnetic cells, however, this approach has drawbacks such as a long magnetization time.

### SUMMARY OF THE DISCLOSURE

The present disclosure provides a magnetic bead removal method, device, and storage medium to solve the problems of immunomagnetic cell escape and low magnetic field utilization in the prior art.

The magnetic bead removal method provided in the present disclosure includes:
placing a liquid bag with a curved flow channel on a magnetic platform, wherein a plane where the curved flow channel is located is above the magnetic platform and parallel to the magnetic platform;
controlling a first flow control assembly to communicate the liquid bag with a sample container, thereby inputting a liquid containing immunomagnetic cells and non-magnetic immune cells into the curved flow channel from the sample container;
performing a flattening operation on the liquid bag to increase a contact surface between a bottom surface of the curved flow channel and the magnetic platform, thereby adsorbing the immunomagnetic cells in the curved flow channel onto the contact surface through the magnetic platform; and
performing a collection operation to collect the non-magnetic immune cells in the curved flow channel into a collection container.

The magnetic bead removal device provided in the presetn disclosure includes a pressing device and a controller for performing the above magnetic bead removal method.

The computer-readable storage medium provided in the present disclosure stores computer-readable instructions, wherein the computer-readable instructions, when executed by a processor, implement the above magnetic bead removal method.

The magnetic bead removal method of the present disclosure includes: placing a liquid bag with a curved flow channel on a magnetic platform, wherein a plane where the curved flow channel is located is above the magnetic platform and parallel to the magnetic platform; controlling a first flow control assembly to communicate the liquid bag with a sample container, thereby inputting a liquid containing immunomagnetic cells and non-magnetic immune cells into the curved flow channel from the sample container; performing a flattening operation on the liquid bag to increase a contact surface between a bottom surface of the curved flow channel and the magnetic platform, thereby adsorbing the immunomagnetic cells in the curved flow channel onto the contact surface through the magnetic platform; and performing a collection operation to collect the non-magnetic immune cells in the curved flow channel into a collection container. The present disclosure utilizes the curved flow channel to achieve a consistent flow velocity of the liquid in the liquid bag, greatly improving the uniformity of the flow velocity and improving the ability of the magnetic platform to fully adsorb the immunomagnetic cells in a uniform flow field, thereby improving the magnetic field utilization efficiency. Moreover, by performing the flattening operation on the liquid bag, the contact surface between the bottom surface of the curved flow channel and the magnetic platform is increased. As a result, the magnetic platform can more fully adsorb the immunomagnetic cells in the curved flow channel through the increased contact surface, avoiding the escape of the immunomagnetic cells and further improving the magnetic field utilization efficiency. Thus, efficient and stable capture of the immunomagnetic cells is achieved with simple operations.

The details of one or more embodiments of the present disclosure are presented in the accompanying drawings and description below, and other features and advantages of the present disclosure will become apparent from the specification, drawings, and claims.

### BRIEF DESCRIPTION OF THE DRAWINGS

To illustrate the technical solutions in this invention or the prior art more clearly, a brief introduction to the drawings required for describing the embodiments or prior art will be provided below. It is evident that the drawings described below are some embodiments of this invention. For those skilled in the art, other drawings can be obtained based on these drawings without requiring creative effort.
FIG. 1 is a flowchart of a magnetic bead removal method according to an embodiment of the present disclosure;
FIG. 2 is a flowchart of a step S30 of the magnetic bead removal method according to an embodiment of the present disclosure;
FIG. 3 is a schematic view of a liquid bag according to an embodiment of the present disclosure;
FIG. 4 is a schematic view of a liquid bag according to another embodiment of the present disclosure;
FIG. 5 is an exploded view of a pressing device according to an embodiment of the present disclosure;
FIG. 6 is an assembly view of the pressing device according to an embodiment of the present disclosure;
FIG. 7 is a schematic diagram of a flow velocity distribution in a large-volume liquid bag in the existing technology; and
FIG. 8 is a schematic view of a computer device according to embodiment of the present disclosure.

The accompanying figures in the manual are labeled as follows:
1, pressing device; 10, magnetic platform; 101, base; 1011, lifting through-hole; 1012, guide through-hole; 102, magnetic block; 20, lifting mechanism; 201, lifting assembly; 2011, first lifting block; 2012, second lifting block; 2013, limit plate; 2014, rotating shaft; 202, lifting rod; 203, ascending driving assembly; 2031, motor; 2032, driving wheel; 2033, driven wheel; 2034, synchronous belt; 2035, shaft; 2036, cam; 2037, mounting bearing; 2038, bracket; 2039, installation hole; 2040, bearing follower; 2041, fixing plate; 2042, sensing block; 2043, photoelectric sensor; 30, covering mechanism; 301, cover plate; 3011, handle; 302, first adsorption portion; 303, second adsorption portion; 40, pressing mechanism; 401, guide shaft; 402, spring; 403, linear bearing; 4011, stopper; 50, protective cover; 60, liquid bag; 61, curved flow channel; 611, straight pipe section; 612, connecting pipe section; 62, liquid inlet; 63, liquid outlet.

### PREFERRED EMBODIMENTS

To make the objectives, technical solutions, and advantages of the present disclosure clearer, the technical solutions of the present disclosure will be clearly and comprehensively described below in conjunction with the accompanying drawings. It is apparent that the described embodiments are a part of the embodiments of the present disclosure, not all embodiments. Based on the embodiments of the present disclosure, all other embodiments obtained by those of ordinary skill in the art without creative effort fall within the scope of protection of the present disclosure.

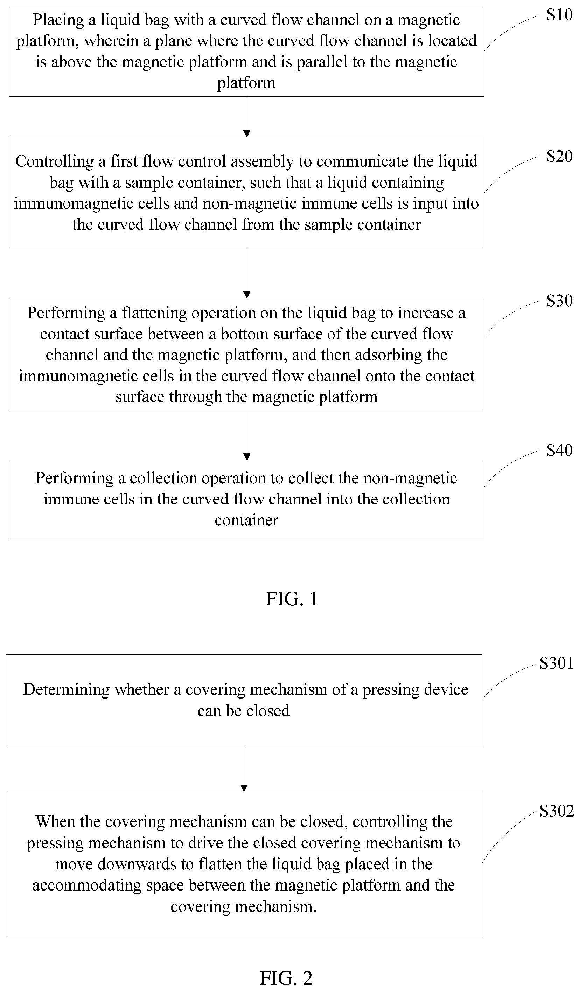

In one embodiment, as shown in FIG. 1, a magnetic bead removal method is provided, including steps as the follows.

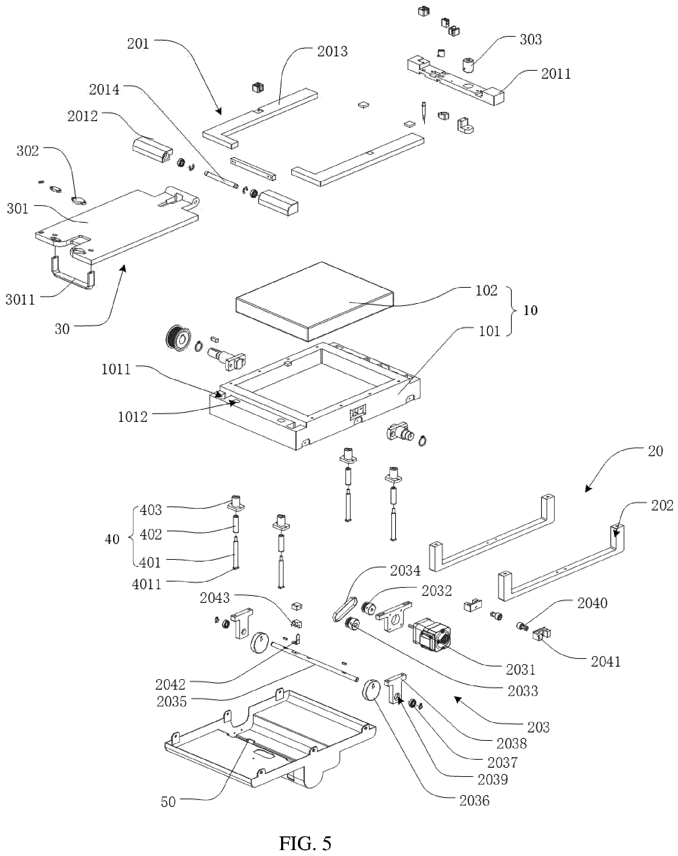

S10, placing a liquid bag 60 with a curved flow channel 61 on a magnetic platform 10, wherein a plane where the curved flow channel 61 is located is above the magnetic platform 10 and is parallel to the magnetic platform 10. The magnetic platform 10 can be formed by electromagnets or permanent magnets. For example, the magnetic platform 10 shown in FIG. 5 may include a base 101 with a lifting through-hole 1011 and a permanent magnet block 102 or an electromagnet arranged on the base 101. An installation slot is formed in the base 101, and the magnetic block 102 is fixedly mounted in the installation slot. The liquid bag 60 with the curved flow channel 61 is made of non-magnetic material and is not magnetically adsorbed, thus, the liquid bag 60 is not affected by the magnetic field of the magnetic platform 10 below the liquid bag 60.

S20, controlling a first flow control assembly (not shown) to communicate the liquid bag 60 with a sample container (not shown), such that a liquid containing immunomagnetic cells and non-magnetic immune cells is input into the curved flow channel 61 from the sample container. The first flow control assembly may include a peristaltic pump that can provide power for a liquid flow, a pinch valve as a switch of the first flow control assembly, and a flow regulating valve to control a flow rate of the liquid flow, or a sensor to monitor the flow rate and a pressure of the liquid flow. The sample container contains the cultured culture medium, which includes immunomagnetic cells and non-magnetic immune cells. The culture medium (i.e. the liquid containing immunomagnetic cells and non-magnetic immune cells in the sample container) contains immunomagnetic cells, as well as non-magnetic immune cells without magnetic beads that are generated by the division of immunomagnetic cells in the culture medium (during the culturing process, magnetic beads do not divide, while cells divide; thus, non-magnetic immune cells with antibodies rather than magnetic beads are generated).

Furthermore, the liquid bag 60 includes a liquid inlet 62 communicating with the sample container through the first flow control assembly and a liquid outlet 63 communicating with a collection container. The curved flow channel 61 is connected between the liquid inlet 62 and the liquid outlet 63. In this embodiment, the culture medium in the sample container can be transported through the liquid inlet 62 to the curved flow channel 61 through the first flow control assembly. Then, under the action of the magnetic platform 10, immunomagnetic cells are adsorbed by the magnetic field of the magnetic platform 10, and non-magnetic immune cells flow out from the liquid outlet 63 of the curved flow channel 61 into the collection container. In this embodiment, the material of the pipes forming the curved flow channel 61 is non-magnetic, such as PVC material, which cannot be affected by the magnetic field of the magnetic platform 10.

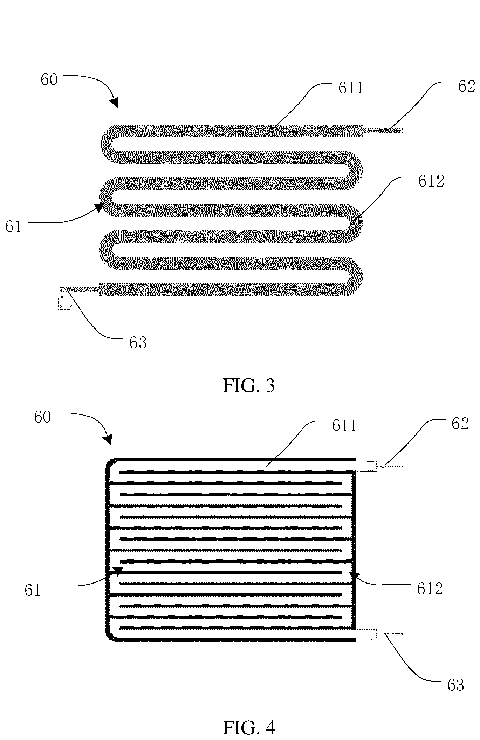

In one embodiment, the curved flow channel 61 includes multiple straight pipe sections 611 arranged in parallel, and a connecting pipe section 612 connected between two adjacent straight pipe sections 611. The connecting pipe section 612 is a bent pipe or a straight pipe at a preset inclination angle with the straight pipe section 611 (the preset inclination angle is set according to requirements, such as the preset inclination angle of 90 degrees shown in FIG. 4, which means that the connecting pipe section 612 is perpendicular to the straight pipe section 611). And/or the curved flow channel 61 includes a spiral pipe section. That is, in this embodiment, the curved flow channel 61 can be a spiral pipe section similar to the mosquito coil in shape, or a combination of the straight pipe sections 611 and the connecting pipe sections 612 as shown in FIG. 3 (the connecting pipe section 612 in FIG. 3 is a bent pipe) and FIG. 4 (the connecting pipe section 612 in FIG. 4 is a straight pipe). Moreover, in some embodiments, the straight pipe section 611, the connecting pipe section 612, and the spiral pipe section can be combined together to form the curved flow channel 61. Understandably, all sections of the curved flow channel are located on the same plane which is parallel to the magnetic platform 10. Such configuration allows the curved flow channel 61 to be affected by the boundary layer, and a liquid flow velocity in the curved flow channel is relatively more uniform, resulting in higher magnetic field utilization efficiency for the magnetic platform 10. On the basis that the curved flow channel 61 can allow the liquid flow velocity to be uniform and improve the magnetic field utilization efficiency, the shape of the curved flow channel 61 can have other shapes according to requirements. Furthermore, a maximum width of the curved flow channel 61 is less than or equal to 50 mm, which allows the curved flow channel 61 to have a better effect on improving magnetic field utilization efficiency.

The liquid bag 60 further includes a buffering space, and the curved flow channel 61 communicates with the liquid outlet 63 through the buffering space. That is, the buffering space can facilitate the buffering and storage of the liquid during the process of flowing from the curved flow channel to the liquid outlet 63, change the direction of the liquid flow, and reduce the flow velocity according to demand. In an embodiment, the curved flow channel 61 is formed by coiling a liquid pipe of 2 * mm (which has an inner diameter of 2 mm, an outer diameter being greater than 2 mm, the liquid pipe can also have other sizes) into a spiral pipe section shaped like a mosquito coil tray, and a circular buffering space is provided at the center of the spiral pipe section. The spiral pipe section communicates with the liquid outlet 63 through the circular buffering space.

S30, performing a flattening operation on the liquid bag 60 to increase a contact surface between a bottom surface of the curved flow channel 61 and the magnetic platform 10, and then adsorbing the immunomagnetic cells in the curved flow channel 61 onto the contact surface through the magnetic platform 10. That is, the contact surface formed by attaching the liquid bag 60 to the magnetic platform 10 increases. In this way, the magnetic platform 10 can closely attach to the immunomagnetic cells in the liquid (culture medium) in the liquid bag 60 through the increased contact surface, such that the liquid can contact the magnetic field of the magnetic platform 10 evenly, and the immunomagnetic cells are adsorbed on the increased contact surface, allowing the magnetic adsorption process to be more complete and avoiding the situation that the immunomagnetic cells are not adsorbed and flow out of the liquid bag 60 from the liquid outlet 63 by following the non-magnetic immune cells. At the same time, due to the increased contact surface of the flattened liquid bag 60, a one-time magnetization adsorption on the magnetic beads in a large volume of cell fluid can be achieved during the magnetic bead sorting, thereby achieving uniform magnetization without controlling the flow velocity and achieving magnetization consistency.

S40, performing a collection operation to collect the non-magnetic immune cells in the curved flow channel 61 into the collection container (not shown). In an embodiment, step S40 includes: turning on a second flow control assembly (not shown) arranged between the collection container and the liquid bag 60 to communicate the liquid bag 60 with the collection container, and transporting the non-magnetic immune cells in the curved flow channel 61 to the collection container through the second flow control assembly to complete the collection of the non-magnetic immune cells, which can then be used for experiments or medical purposes. That is, in this embodiment, both the sample container and the collection container communicate with the liquid bag 60. The first flow control assembly is arranged between the sample container and the liquid bag 60, and the second flow control assembly is arranged between the collection container and the liquid bag 60. The second flow control assembly may include a peristaltic pump that can provide power for the liquid flow, a pinch valve as a switch of the second flow control assembly, a flow control valve for controlling the flow rate of the liquid flow, or a sensor for monitoring the flow velocity and the pressure of the liquid flow. In the present disclosure, only one of the first flow control assembly and the second flow control assembly can be arranged to control the flow parameters of the liquid in the liquid bag 60, such as the flow velocity, the flow rate, and the pressure. In other embodiments, both the first flow control assembly and the second flow control assembly can be arranged simultaneously.

The present disclosure utilizes the curved flow channel 61 to achieve a consistent flow velocity of the liquid in the liquid bag 60, greatly improving the uniformity of the flow velocity and improving the ability of the magnetic platform 10 to fully adsorb the immunomagnetic cells in a uniform flow field, thereby improving the magnetic field utilization efficiency. Moreover, by performing the flattening operation on the liquid bag 60, the contact surface between the bottom surface of the curved flow channel 61 and the magnetic platform 10 is increased. As a result, the magnetic platform 10 can more fully adsorb the immunomagnetic cells in the curved flow channel 61 through the increased contact surface, avoiding the escape of immunomagnetic cells and further improving the magnetic field utilization efficiency, achieving efficient and stable capture of immunomagnetic cells with simple operations.

In one embodiment, as shown in FIG. 2, step S30 includes steps as follows.

S301, determining whether a covering mechanism 30 of a pressing device 1 can be closed. As shown in FIGS. 5 and 6, the pressing device 1 includes the covering mechanism 30 arranged on the magnetic platform 10 and a pressing mechanism 40 connected to the covering mechanism 30. When the covering mechanism 30 is closed, the liquid bag 60 is located in an accommodating space between the covering mechanism 30 and the magnetic platform 10. The covering mechanism 30 is arranged on the magnetic platform 10, and can close or open the accommodating space (when the liquid bag 60 placed in the accommodating space expands and protrudes relatively high from the accommodating space, the covering mechanism 30 is pushed up by the liquid bag 60 and cannot be closed, and forcible closing of the covering mechanism 30 may crush the liquid bag 60 or damage the immunomagnetic cells or non-nuclear immune cells inside the liquid bag 60). When not interfered by other external forces, the covering mechanism 30 can move downwards with the downward pull of the pressing mechanism 40, thereby allowing the accommodating space between the magnetic platform 10 and the covering mechanism 30 for placing the liquid bag 60 to be adjustable.

Step 302, when the covering mechanism 30 can be closed, controlling the pressing mechanism 40 to drive the closed covering mechanism 30 to move downwards to flatten the liquid bag 60 placed in the accommodating space between the magnetic platform 10 and the covering mechanism 30. It can be understood that when the covering mechanism 30 can be closed, the pressing mechanism 40 can be used to drive the closed covering mechanism 30 to move downwards and evenly flatten the liquid bag 60, such that the contact surface formed by attaching the liquid bag 60 to the magnetic platform 10 increases, and the liquid in the liquid bag 60 can be evenly distributed on the magnetic platform 10 through the contact surface and in contact with the magnetic field of the magnetic platform 10.

Furthermore, as shown in FIGS. 5 and 6, the pressing device 1 further includes a lifting mechanism 20 connected to the covering mechanism 30. After step S301, that is, after determining whether the covering mechanism 30 of the pressing device 1 can be closed, step 30 further includes steps as follows.

When the covering mechanism 30 is pushed up by the expanded liquid bag 60 and cannot be closed, controlling the lifting mechanism 20 to drive the covering mechanism 30 to ascend, thereby increasing the accommodating space between the magnetic platform 10 and the covering mechanism 30. In this embodiment, after placing the liquid bag 60 in the accommodating space, since the liquid bag 60 expands and protrudes from the accommodating space, the covering mechanism 30 is pushed up and opened by the liquid bag 60 placed in the accommodating space, thus the covering mechanism 30 cannot be closed. At this time, the lifting mechanism 20 is turned on to drive the covering mechanism 30 to ascend. As a result, the distance between the magnetic platform 10 and the covering mechanism 30 increases, and the accommodating space increases with the increased distance.

After the covering mechanism 30 ascends to a preset height at which the covering mechanism 30 can be closed, closing the covering mechanism 30. That is, when the covering mechanism 30 ascends to the preset height, the accommodating space between the covering mechanism 30 and the magnetic platform 10 is already large enough, and the covering mechanism 30 is no longer pushed up by the liquid bag 60 and can be closed normally.

Controlling the pressing mechanism 40 to drive the closed covering mechanism 30 to move downwards, thereby flattening the liquid bag 60 placed in the accommodating space. That is, after the covering mechanism 30 is closed, the closed covering mechanism 30 can be driven to move downwards by the pressing mechanism 40 to evenly flatten the liquid bag 60, such that the contact surface formed by attaching the liquid bag 60 to the magnetic platform 10 increases, and the liquid in the liquid bag 60 can be evenly distributed on the magnetic platform 10 through the contact surface and in contact with the magnetic platform 10.

In one embodiment, as shown in FIGS. 5 and 6, the lifting through-hole 1011 is formed in the magnetic platform 10. The lifting mechanism 20 includes a lifting assembly 201 arranged at a top of the magnetic platform 10, a lifting rod 202 with a top thereof fixedly connected to the lifting assembly 201 by passing through the lifting through-hole 1011, and an ascending driving assembly 203 arranged below the magnetic platform 10 and connected to one end of the lifting rod 202 away from the lifting assembly 201. The covering mechanism 30 includes a cover plate 301 rotatably connected to the lifting assembly 201. A shape of the cover plate 301 can be set according to the shape of the magnetic platform 10. Furthermore, the covering mechanism 30 further includes a handle 3011 arranged on the cover plate 301, such that the cover plate 301 can be lifted and closed by users through the handle 3011. A shape of the lifting assembly 201 can be set according to the requirements. For example, the lifting assembly 201 can be a frame structure arranged on the top of the magnetic platform 10 (or formed by multiple separated components which can can move up and down simultaneously with the lifting rod 202), and can be driven by the ascending driving assembly 203 to move up and down. Understandably, the ascending driving assembly 203, as the ascending driving force source for the lifting assembly 201, can include a motor 2031, a cylinder, etc. In one embodiment, as shown in FIGS. 5 and 6, the lifting assembly 201 includes a first lifting block 2011 arranged at a first end of the magnetic platform 10, two spaced-apart second lifting blocks 2012 arranged on a second end of the magnetic platform 10 away from the first end, a limit plate 2013 connected between the first lifting block 2011 and the corresponding second lifting block 2012, and a rotating shaft 2014 connected between the two second lifting blocks 2012. The cover plate 301 is rotatably connected to the rotating shaft 2014, and a second adsorption portion 303 as shown in FIG. 5 is arranged on the first lifting block 2011. In this embodiment, the lifting assembly 201 includes two limit plates 2013 arranged in parallel to each other on opposite sides of the magnetic platform 10. In this way, the first lifting block 2011, the two second lifting blocks 2012, and the limit plates 2013 are enclosed to form a limit frame to prevent the liquid bag 60 from escaping from the side of the accommodating space. The limit frame (which is the main component of the lifting assembly 201) can move up and down synchronously with the cover plate 301.

Furthermore, the controlling the lifting mechanism 20 to drive the covering mechanism 30 to ascend, thereby increasing the accommodating space between the magnetic platform 10 and the covering mechanism 30 includes: turning on the ascending driving assembly 203 to drive the lifting rod 202 and thus to drive the lifting assembly 201 and the cover plate 301 to slide up along the lifting through-hole 1011 through the lifting rod 202, thereby increasing the accommodating space between the magnetic platform 10 and the cover plate 301 for placing the liquid bag 60. That is, the covering mechanism 30 is rotatably arranged on the lifting assembly 201 and moves along with the up and down movement of the lifting assembly 201. The ascending driving assembly 203 can drive the lifting assembly 201 to move up along the lifting through-hole 1011 through the lifting rod 202. In this way, the cover plate 301 can be driven to move up, thereby increasing the accommodating space between the magnetic platform 10 and the cover plate 301 for placing the liquid bag 60, and facilitating the closing of the cover plate 301.

Furthermore, as shown in FIGS. 5 and 6, the covering mechanism 30 further includes a first adsorption portion 302 arranged on the cover plate 301, and a second adsorption portion 303 arranged on the lifting assembly 201 at a position opposite to the first adsorption portion 302. The cover plate 301 is closed to the lifting assembly 201 through the adsorption of the first adsorption portion 302 and the second adsorption portion 303. At least one of the first adsorption portion 302 and the second adsorption portion 303 is a magnet; in some embodiments, both the first adsorption portion 302 and the second adsorption portion 303 can be magnets; in other embodiments, one of the first adsorption portion 302 and the second adsorption portion 303 is a magnet, and the other can be a metal with magnetic adsorption characteristics. The number, combination, specific shape and size of the first adsorption portion 302 and the second adsorption portion 303 can be set according to actual needs, which are not limited here. The magnet in the present disclosure can also refer to an electromagnet. In an embodiment, the second adsorption portion 303 is an electromagnet, and the first adsorption portion 302 is an iron block. The electromagnet of the second adsorption portion 303 can be energized to remove magnetism. At this time, there is no magnetic adsorption between the electromagnet of the first adsorption portion 302 (iron block) on the cover plate 301 and the second adsorption portion 303, thus, the first adsorption portion 302 and the second adsorption portion 303 are in a separated state. In this way, the cover plate 301 can be opened. After the second adsorption portion 303 is powered off, the electromagnet acting as the second adsorption portion 303 generates magnetism. At this time, in the absence of external force, when the iron block on the cover plate 301 is within the magnetic adsorption range of the electromagnet, the first adsorption portion 302 of the cover plate 301 is adsorbed by the second adsorption portion 303, thus completing the closing of the cover plate 301.

In one embodiment, as shown in FIGS. 5 and 6, the ascending driving assembly 203 includes a motor 2031, a driving wheel 2032, a driven wheel 2033, a synchronous belt 2034, a rotating shaft 2035, a cam 2036, a mounting bearing 2037, and a bracket 2038 with an installation hole 2039. The bracket 2038 is arranged at the bottom of the magnetic platform 10, the rotating shaft 2035 is arranged in the installation hole 2039 through the mounting bearing 2037, the cam 2036 is fixedly arranged on the rotating shaft 2035, the driving wheel 2032 is arranged on an output shaft of the motor 2031, the synchronous belt 2034 is sleeved on the driving wheel 2032 and the driven wheel 2033, the driven wheel 2033 is fixedly arranged on the rotating shaft 2035, and a top of the cam 2036 abuts the lifting rod 202. As shown in FIG. 5, the motor 2031 can be fixed on the magnetic platform 10 through a fixed block. In an embodiment, the ascending driving assembly 203 includes two cams 2036, two brackets 2038, two mounting bearings 2037, and two lifting rods 202; and the two cams 2036, two brackets 2038, and two mounting bearings 2037 are symmetrically arranged on the rotating shaft 2035. The two lifting rods 202 are arranged in parallel, and top ends of the two lifting rods 202 are connected to the lifting assembly 201. The two cams 2036 respectively abut bottom ends of the two lifting rods 202.

Furthermore, the ascending driving assembly 203 drives the lifting rod 202 to drive the lifting assembly 201 and the cover plate 301 to slide up along the lifting through-hole 1011, including: turning on the motor 2031 to drive the cam 2036 to sequentially rotate through the driving wheel 2032, the synchronous belt 2034, the driven wheel 2033, and the rotating shaft 2035, and driving the lifting assembly 201 and the cover plate 301 to slide up along the lifting through-hole 1011 through the lifting rod 202 by a rotation of the cam 2036. That is, after the motor 2031 starts to rotate, the motor 2031 drives the driving wheel 2032 to rotate, and then drives the driven wheel 2033 to rotate through the synchronous belt 2034. The rotation of the driven wheel 2033 drives the rotating shaft 2035 and the two cams 2036 arranged on the rotating shaft 2035 to rotate. At this time, the lifting rod 202 abutting the cam 2036 moves up with the rotation of the cam 2036, which drives the lifting assembly 201 and the cover plate 301 to ascend together. At this time, if the second adsorption portion 303 is an electromagnet as described in the above embodiment, the electromagnet of the second adsorption portion 303 is powered off; during the process, the cover plate 301 ascends as the lifting assembly 201 ascends; when the iron block of the first adsorption portion 302 is adsorbed to the electromagnet of the second adsorption portion 303, it is confirmed that the cover plate 301 is closed.

Furthermore, as shown in FIG. 5, the ascending driving assembly 203 further includes an induction block 2042 arranged on the rotating shaft 2035 and a photoelectric sensor 2043 arranged on the magnetic platform 10. When the photoelectric sensor 2043 senses that the rotating shaft 2035 drives the induction block 2042 to rotate to a position facing the photoelectric sensor 2043, the photoelectric sensor 2043 determines that the cover plate 301 reaches the preset height. The preset height refers to the largest height which the lifting assembly 201 and the cover plate 301 can be driven to reach by the lifting rod 202. The preset height can be set according to the specific size of the liquid bag 60, and can be achieved by changing the size and shape of the cam 2036 according to needs.

In one embodiment, as shown in FIG. 5, the ascending driving assembly 203 further includes a bearing follower 2040 and a fixing plate 2041. The bearing follower 2040 is mounted at the bottom end of the lifting rod 202 through the fixing plate 2041, and the cam 2036 abuts the bearing follower 2040. That is, the bearing follower 2040 can better transmit the lifting force generated by the rotation of the cam 2036 to the lifting rod 202, thereby allowing the lifting rod 202 to move up more stably and controllably.

In one embodiment, as shown in FIGS. 5 and 6, a guide through-hole 1012 is formed in the magnetic platform 10. The pressing mechanism 40 includes a guide shaft 401, a spring 402, and a linear bearing 403 arranged in the guide through-hole 1012. A stopper 4011 is arranged on a bottom end of the guide shaft 401, and a top end of the guide shaft 401 passes through the linear bearing 403 to be connected to the lifting assembly 201. The spring 402 is sleeved on the guide shaft 401, and both ends of the spring 402 respectively abut the linear bearing 403 and the stopper 4011. Furthermore, in step 302, the controlling the pressing mechanism 40 to drive the closed covering mechanism 30 to move downwards includes: turning off the ascending driving assembly 203, thereby driving the guide shaft 401 to move downwards along the guide through-hole 1012 through an elastic force of the spring 402, and thus driving the lifting assembly 201 and the cover plate 301 to move downwards. In this embodiment, the ascending driving assembly 203 with the cam 2036 can only drive the lifting rod 202 to ascend, and does not have a downward pulling force to pull down the lifting assembly 201. Thus, the above-mentioned pressing mechanism 40 is provided, and the spring 402 is in a compressed state. After the motor 2031 of the ascending driving assembly 203 is turned off (the motor 2031 is reset), the spring 402 restores to its original state and applies a downward pulling force to the lifting assembly 201 through the guide shaft 401 (the spring 402 drives the guide shaft 401 to move downwards along the guide through-hole 1012), thereby driving the lifting assembly 201 and the cover plate 301 to move downwards. Understandably, if the guide shaft 401, the spring 402, and the linear bearing 403 are used as a pressing assembly, the pressing mechanism 40 can include multiple pressing assemblies arranged on the magnetic platform 10, such as four pressing assemblies arranged at the four corners of the magnetic platform 10. In this way, when the four springs 402 of the four pressing assemblies simultaneously apply a downward pulling force to the lifting assembly 201, the cover plate 301 presses the liquid bag 60 towards the direction of the magnetic platform 10 as the lifting assembly 201 moves downwards, gradually flattening the liquid bag 60 and evenly distributing the liquid on the magnetic platform 10. With such configuration, the liquid bag 60 is flattened and pressed down by the spring 402 instead of the motor 2031. On the one hand, the liquid bag 60 can be evenly flattened and attached to the magnetic platform 10 by the spring 402. On the other hand, the elastic force of the spring 402 is uniform and gentle, which does not crush the cells inside the liquid bag 60.

In one embodiment, as shown in FIGS. 5 and 6, the magnetic sorting and the pressing device 1 further includes a protective cover 50 mounted at the bottom of the magnetic platform 10, and an installation space is formed between the protective cover 50 and the magnetic platform 10. The ascending driving assembly 203 is arranged in the installation space. The protective cover 50 can accommodate and protect various components in the installation space, such as the ascending driving assembly 203, the pressing mechanism 40, or other electronic components. In an embodiment, the protective cover 50 can be made of sheet metal, which has a more sturdy and reliable structure.

It should be understood that the size of the sequence numbers of each step in the above embodiments does not imply the order of execution. The order of execution of each process should be determined by its function and internal logic, and should not constitute any limitation on the implementation process of the present disclosure.

The present disclosure also provides a magnetic bead removal device, including the pressing device 1 and a controller for performing the above-mentioned magnetic bead removal method, wherein the controller is connected to the pressing device 1.

The controller is used to perform the following steps:
placing a liquid bag with a curved flow channel on a magnetic platform, wherein a plane where the curved flow channel is located is above the magnetic platform and parallel to the magnetic platform;
controlling a first flow control assembly to communicate the liquid bag with a sample container, thereby inputting a liquid containing immunomagnetic cells and non-magnetic immune cells into the curved flow channel from the sample container;
performing a flattening operation on the liquid bag to increase a contact surface between a bottom surface of the curved flow channel and the magnetic platform, thereby adsorbing the immunomagnetic cells in the curved flow channel onto the contact surface through the magnetic platform; and
performing a collection operation to collect the non-magnetic immune cells in the curved flow channel into a collection container.

In an embodiment, the liquid bag includes a liquid inlet communicating with the sample container through the first flow control assembly and a liquid outlet communicating with the collection container; and the curved flow channel is connected between the liquid inlet and the liquid outlet of the liquid bag.

In an embodiment, the curved flow channel includes multiple straight pipe sections arranged in parallel, and a connecting pipe section connected between two adjacent straight pipe sections; the connecting pipe section is a bent pipe or a straight pipe arranged at a predetermined inclination angle with the straight pipe section; and/or
the curved flow channel includes a spiral pipe section.

In an embodiment, a maximum width of the curved flow channel is less than or equal to 50 mm; and/or
the liquid bag further includes a buffering space, and the curved flow channel communicates with the liquid outlet of the liquid bag through the buffering space.

In an embodiment, the performing a flattening operation on the liquid bag includes:
determining whether a covering mechanism of a pressing device is capable of being closed, wherein the pressing device includes the covering mechanism arranged on the magnetic platform and a pressing mechanism connected to the covering mechanism; when the covering mechanism is closed, the liquid bag is located in an accommodating space between the covering mechanism and the magnetic platform; and
when the covering mechanism is capable of being closed, controlling the pressing device to drive the closed covering mechanism to move downwards, thereby flattening the liquid bag placed in the accommodating space between the magnetic platform and the covering mechanism.

In an embodiment, the pressing device further includes a lifting mechanism connected to the covering mechanism;
after the determining whether a covering mechanism of a pressing device is capable of being closed, the controller is used to further perform the following steps:
when the covering mechanism is pushed up by the expanded liquid bag and incapable of being closed, controlling the lifting mechanism to drive the covering mechanism to ascend, thereby increasing the accommodating space between the magnetic platform and the covering mechanism;
after the covering mechanism ascends to a preset height at which the covering mechanism is capable of being closed, closing the covering mechanism; and
controlling the pressing device to drive the closed covering mechanism to move downwards to flatten the liquid bag placed in the accommodating space.

In an embodiment, a lifting through-hole is formed in the magnetic platform, and the lifting mechanism includes a lifting assembly arranged at a top of the magnetic platform, a lifting rod with a top thereof fixedly connected to the lifting assembly by passing through the lifting through-hole, and an ascending driving assembly arranged below the magnetic platform and connected to one end of the lifting rod away from the lifting assembly; the covering mechanism includes a cover plate rotatably connected to the lifting assembly;
the controlling the lifting mechanism drives the covering mechanism to ascend, thereby increasing the accommodating space between the magnetic platform and the covering mechanism, includes:
turning on the ascending driving assembly to drive the lifting rod and thus to drive the lifting assembly and the cover plate to slide up along the lifting through-hole through the lifting rod, thereby increasing the accommodating space between the magnetic platform and the cover plate for placing the liquid bag.

In an embodiment, the ascending driving assembly includes a motor, a driving wheel, a driven wheel, a synchronous belt, a rotating shaft, a cam, a mounting bearing, and a bracket with an installation hole; the bracket is arranged at a bottom of the magnetic platform, the rotating shaft is arranged in the installation hole through the mounting bearing, the cam is fixedly arranged on the rotating shaft, the driving wheel is arranged on an output shaft of the motor, the synchronous belt is sleeved on the driving wheel and the driven wheel, the driven wheel is fixedly arranged on the rotating shaft, and a top of the cam abuts the lifting rod;
the driving the lifting assembly and the cover plate to slide up along the lifting through-hole through the ascending driving assembly includes:
turning on the motor to drive the cam to sequentially rotate through the driving wheel, the synchronous belt, the driven wheel, and the rotating shaft, and driving the lifting assembly and the cover plate to slide up along the lifting through-hole through the lifting rod by a rotation of the cam.

In an embodiment, the performing a collection operation to collect non-magnetic immune cells in the curved flow channel into a collection container includes:
turning on a second flow control assembly arranged between the collection container and the liquid bag, such that the liquid bag is connected to the collection container, and transporting the non-magnetic immune cells in the curved flow channel to the collection container through the second flow control assembly.

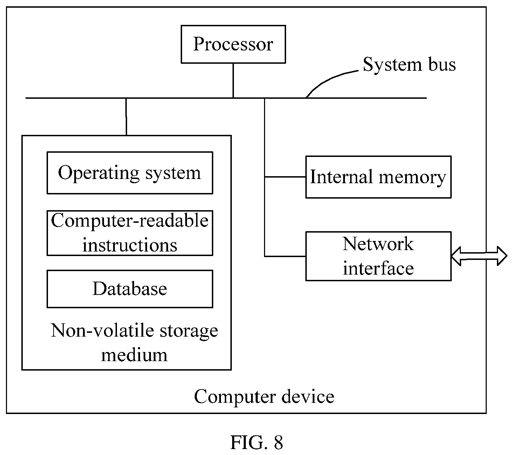

For more specific limitations on the pressing device 1 and the controller of the magnetic bead removal device, please refer to the previous section on the limitations of magnetic bead removal method, which is not repeated here. The various modules in the above controller can be fully or partially implemented through software, hardware, and their combinations. The above modules can be embedded in hardware form or independent of the processor in the computer device, or stored in software form in the memory of the computer device, such that the processor can call and execute the corresponding operations of the above modules. Understandably, the controller can be considered as one or more computer devices; as shown in FIG. 8, the computer device can include a processor, a memory, a network interface, and a database connected through a system bus. The processor of the computer device is used to provide computing and control capabilities. The memory of the computer device includes a a computer-readable storage medium and an internal memory. The computer-readable storage medium stores an operating system, computer-readable instructions, and a database. The internal memory provides an environment for the execution of the operating system and the computer-readable instructions of the computer-readable storage medium. The database of the computer device is used to store the data used for the magnetic bead removal method in the above embodiments. The network interface of the computer device is used to communicate with external terminals through a network connection. The computer-readable instruction is executed by the processor to implement the above magnetic bead removal method. The computer-readable storage medium provided in this embodiment include a non-volatile computer-readable storage medium and a volatile computer-readable storage medium.

In one embodiment, one or more readable storage media storing computer-readable instructions are provided, and the computer-readable storage media provided in this embodiment include non-volatile computer-readable storage media and volatile computer-readable storage media. The computer-readable storage medium stores computer-computer-readable instructions which, when executed by one or more processors, enable one or more processors to implement the magnetic bead removal method described above.

In an embodiment, when being executed by one or more processors, the computer-readable instructions cause the one or more processors to implement the following steps:
placing a liquid bag with a curved flow channel on a magnetic platform, wherein a plane where the curved flow channel is located is above the magnetic platform and parallel to the magnetic platform;
controlling a first flow control assembly to communicate the liquid bag with a sample container, thereby inputting a liquid containing immunomagnetic cells and non-magnetic immune cells into the curved flow channel from the sample container;
performing a flattening operation on the liquid bag to increase a contact surface between a bottom surface of the curved flow channel and the magnetic platform, thereby adsorbing the immunomagnetic cells in the curved flow channel onto the contact surface through the magnetic platform; and
performing a collection operation to collect the non-magnetic immune cells in the curved flow channel into a collection container.

In an embodiment, the liquid bag includes a liquid inlet communicating with the sample container through the first flow control assembly and a liquid outlet communicating with the collection container; and the curved flow channel is connected between the liquid inlet and the liquid outlet of the liquid bag.

In an embodiment, the curved flow channel includes multiple straight pipe sections arranged in parallel, and a connecting pipe section connected between two adjacent straight pipe sections; the connecting pipe section is a bent pipe or a straight pipe arranged at a predetermined inclination angle with the straight pipe section; and/or
the curved flow channel includes a spiral pipe section.

In an embodiment, a maximum width of the curved flow channel is less than or equal to 50 mm; and/or
the liquid bag further includes a buffering space, and the curved flow channel communicates with the liquid outlet of the liquid bag through the buffering space.

In an embodiment, the performing a flattening operation on the liquid bag includes:
determining whether a covering mechanism of a pressing device is capable of being closed, wherein the pressing device includes the covering mechanism arranged on the magnetic platform and a pressing mechanism connected to the covering mechanism; when the covering mechanism is closed, the liquid bag is located in an accommodating space between the covering mechanism and the magnetic platform; and
when the covering mechanism is capable of being closed, controlling the pressing device to drive the closed covering mechanism to move downwards, thereby flattening the liquid bag placed in the accommodating space between the magnetic platform and the covering mechanism.

In an embodiment, the pressing device further includes a lifting mechanism connected to the covering mechanism;
after the determining whether a covering mechanism of a pressing device is capable of being closed, the computer-readable instructuions, when being executed by one or more processors, cause the one or more processors to further implement the following steps:
when the covering mechanism is pushed up by the expanded liquid bag and incapable of being closed, controlling the lifting mechanism to drive the covering mechanism to ascend, thereby increasing the accommodating space between the magnetic platform and the covering mechanism;
after the covering mechanism ascends to a preset height at which the covering mechanism is capable of being closed, closing the covering mechanism; and
controlling the pressing device to drive the closed covering mechanism to move downwards to flatten the liquid bag placed in the accommodating space.

In an embodiment, a lifting through-hole is formed in the magnetic platform, and the lifting mechanism includes a lifting assembly arranged at a top of the magnetic platform, a lifting rod with a top thereof fixedly connected to the lifting assembly by passing through the lifting through-hole, and an ascending driving assembly arranged below the magnetic platform and connected to one end of the lifting rod away from the lifting assembly; the covering mechanism includes a cover plate rotatably connected to the lifting assembly;
the controlling the lifting mechanism drives the covering mechanism to ascend, thereby increasing the accommodating space between the magnetic platform and the covering mechanism, includes:
turning on the ascending driving assembly to drive the lifting rod and thus to drive the lifting assembly and the cover plate to slide up along the lifting through-hole through the lifting rod, thereby increasing the accommodating space between the magnetic platform and the cover plate for placing the liquid bag.

In an embodiment, the ascending driving assembly includes a motor, a driving wheel, a driven wheel, a synchronous belt, a rotating shaft, a cam, a mounting bearing, and a bracket with an installation hole; the bracket is arranged at a bottom of the magnetic platform, the rotating shaft is arranged in the installation hole through the mounting bearing, the cam is fixedly arranged on the rotating shaft, the driving wheel is arranged on an output shaft of the motor, the synchronous belt is sleeved on the driving wheel and the driven wheel, the driven wheel is fixedly arranged on the rotating shaft, and a top of the cam abuts the lifting rod;
the driving the lifting assembly and the cover plate to slide up along the lifting through-hole through the ascending driving assembly includes:
turning on the motor to drive the cam to sequentially rotate through the driving wheel, the synchronous belt, the driven wheel, and the rotating shaft, and driving the lifting assembly and the cover plate to slide up along the lifting through-hole through the lifting rod by a rotation of the cam.

In an embodiment, the performing a collection operation to collect non-magnetic immune cells in the curved flow channel into a collection container includes:
turning on a second flow control assembly arranged between the collection container and the liquid bag, such that the liquid bag is connected to the collection container, and transporting the non-magnetic immune cells in the curved flow channel to the collection container through the second flow control assembly

For more specific limitations on readable storage media, please refer to the limitations on magnetic bead removal methods mentioned above, which will not be repeated here.

Those skilled in the art may understand that all or some of the processes of the method in the above embodiments may be implemented by instructing related hardware by using computer-readable instructions. The computer-readable instructions may be stored in a non-volatile computer-readable storage medium or a volatile computer-readable storage medium. When the computer-readable instructions are executed, the processes in the above method embodiments may be implemented. Any reference to the memory, storage, database, or other media used in the embodiments provided in the present disclosure may include a non-volatile and/or volatile memory. The non-volatile memory may include a read-only memory (ROM), a programmable ROM (PROM), an electrically programmable ROM (EPROM), an electrically erasable programmable ROM (EEPROM), or a flash memory. The volatile memory may include a random access memory (RAM) or an external cache memory. By way of illustration instead of limitation, the RAM is available in a plurality of forms, such as a static RAM (SRAM), a dynamic RAM (DRAM), a synchronous DRAM (SDRAM), a double data rate SDRAM (DDRSDRAM), an enhanced SDRAM (ESDRAM), a synchlink (Synchlink) DRAM (SLDRAM), a Rambus (Rambus) direct RAM (RDRAM), a direct Rambus dynamic RAM (DRDRAM), and a Rambus dynamic RAM (RDRAM).

Those skilled in the art may clearly understand that, for convenience and brevity of description, only the division of the above functional units or modules is used as an example for description. In actual applications, the above functions may be allocated to different functional units or modules for implementation as required, that is, an internal structure of the device is divided into different functional units or modules to implement all or some of the functions described above.

The above descriptions are only optional embodiments of the application, and do not limit the scope of the patents of the present disclosure. All the equivalent structural transformations made by the content of the specification and drawings of the present disclosure under the creative concept of the present disclosure, or directly/indirectly used in other related technical fields are all comprised in the protection scope of the patents of the present disclosure.

## Claims

1. A magnetic bead removal method, comprising:
placing a liquid bag with a curved flow channel on a magnetic platform, wherein a plane where the curved flow channel is located is above the magnetic platform and parallel to the magnetic platform;
controlling a first flow control assembly to communicate the liquid bag with a sample container, thereby inputting a liquid containing immunomagnetic cells and non-magnetic immune cells into the curved flow channel from the sample container;
performing a flattening operation on the liquid bag to increase a contact surface between a bottom surface of the curved flow channel and the magnetic platform, thereby adsorbing the immunomagnetic cells in the curved flow channel onto the contact surface through the magnetic platform; and
performing a collection operation to collect the non-magnetic immune cells in the curved flow channel into a collection container.

2. The magnetic bead removal method according to claim 1, wherein the liquid bag comprises a liquid inlet communicating with the sample container through the first flow control assembly and a liquid outlet communicating with the collection container; and the curved flow channel is connected between the liquid inlet and the liquid outlet of the liquid bag.

3. The magnetic bead removal method according to claim 2, wherein the curved flow channel comprises multiple straight pipe sections arranged in parallel, and a connecting pipe section connected between two adjacent straight pipe sections; the connecting pipe section is a bent pipe or a straight pipe arranged at a predetermined inclination angle with the straight pipe section; and/or
the curved flow channel comprises a spiral pipe section.

4. The magnetic bead removal method according to claim 3, wherein a maximum width of the curved flow channel is less than or equal to 50 mm; and/or
the liquid bag further comprises a buffering space, and the curved flow channel communicates with the liquid outlet of the liquid bag through the buffering space.

5. The magnetic bead removal method according to claim 1, wherein the performing a flattening operation on the liquid bag comprises:
determining whether a covering mechanism of a pressing device is capable of being closed, wherein the pressing device comprises the covering mechanism arranged on the magnetic platform and a pressing mechanism connected to the covering mechanism; when the covering mechanism is closed, the liquid bag is located in an accommodating space between the covering mechanism and the magnetic platform; and
when the covering mechanism is capable of being closed, controlling the pressing device to drive the closed covering mechanism to move downwards, thereby flattening the liquid bag placed in the accommodating space between the magnetic platform and the covering mechanism.

6. The magnetic bead removal method according to claim 5, wherein the pressing device further comprises a lifting mechanism connected to the covering mechanism;
after the determining whether a covering mechanism of a pressing device is capable of being closed, the magnetic bead removal method further comprises:
when the covering mechanism is pushed up by the expanded liquid bag and incapable of being closed, controlling the lifting mechanism to drive the covering mechanism to ascend, thereby increasing the accommodating space between the magnetic platform and the covering mechanism;
after the covering mechanism ascends to a preset height at which the covering mechanism is capable of being closed, closing the covering mechanism; and
controlling the pressing device to drive the closed covering mechanism to move downwards to flatten the liquid bag placed in the accommodating space.

7. The magnetic bead removal method according to claim 6, wherein a lifting through-hole is formed in the magnetic platform, and the lifting mechanism comprises a lifting assembly arranged at a top of the magnetic platform, a lifting rod with a top thereof fixedly connected to the lifting assembly by passing through the lifting through-hole, and an ascending driving assembly arranged below the magnetic platform and connected to one end of the lifting rod away from the lifting assembly; the covering mechanism comprises a cover plate rotatably connected to the lifting assembly;
the controlling the lifting mechanism drives the covering mechanism to ascend, thereby increasing the accommodating space between the magnetic platform and the covering mechanism, comprises:
turning on the ascending driving assembly to drive the lifting rod and thus to drive the lifting assembly and the cover plate to slide up along the lifting through-hole through the lifting rod, thereby increasing the accommodating space between the magnetic platform and the cover plate for placing the liquid bag.

8. The magnetic bead removal method according to claim 7, wherein the ascending driving assembly comprises a motor, a driving wheel, a driven wheel, a synchronous belt, a rotating shaft, a cam, a mounting bearing, and a bracket with an installation hole; the bracket is arranged at a bottom of the magnetic platform, the rotating shaft is arranged in the installation hole through the mounting bearing, the cam is fixedly arranged on the rotating shaft, the driving wheel is arranged on an output shaft of the motor, the synchronous belt is sleeved on the driving wheel and the driven wheel, the driven wheel is fixedly arranged on the rotating shaft, and a top of the cam abuts the lifting rod;
the driving the lifting assembly and the cover plate to slide up along the lifting through-hole comprises:
turning on the motor to drive the cam to sequentially rotate through the driving wheel, the synchronous belt, the driven wheel, and the rotating shaft, and driving the lifting assembly and the cover plate to slide up along the lifting through-hole through the lifting rod by a rotation of the cam.

9. The magnetic bead removal method according to claim 1, wherein the performing a collection operation to collect non-magnetic immune cells in the curved flow channel into a collection container comprises:
turning on a second flow control assembly arranged between the collection container and the liquid bag, such that the liquid bag communicates with the collection container, and transporting the non-magnetic immune cells in the curved flow channel to the collection container through the second flow control assembly.

10. A magnetic bead removal device comprising a pressing device and a controller for performing the magnetic bead removal method according to any one of claims 1 to 9, wherein the controller is connected to the pressing device.

11. A computer-readable storage medium storing computer-readable instructions, wherein the computer-readable instructions, when executed by a processor, implement the magnetic bead removal method according to any one of claims 1 to 9.
